# EUROPEAN PATENT SPECIFICATION

(11) **EP 2 307 538 B1**
(45) Date of publication and mention of the grant of the patent: **21.10.2015**
(21) Application number: 09797474.5
(22) Date of filing: 09.07.2009
(51) Int. Cl.: C12N 5/071

(54) **SCALABLE PROCESS FOR CULTURING PER.C6 CELLS AND PRODUCING PRODUCTS THEREFROM**
SKALIERBARES VERFAHREN ZUR KULTIVIERUNG PER.C6 ZELLEN UND HERSTELLUNG VON PRODUKTEN DARAUS
PROCESSUS EXTENSIBLE POUR CULTIVER DES CELLULES PER.C6 ET LEURS PRODUITS DE PRODUCTION

(30) Priority: 15.07.2008 EP 08160392; 15.07.2008 US 134992
(43) Date of publication of application: 13.04.2011
(73) Proprietor: Crucell Holland B.V., 2333 CN Leiden (NL)
(72) Inventor: VERSCHUUR, Maartje, NL-2333 CN Leiden (NL); YALLOP, Christopher, Adam, NL-2333 CN Leiden (NL); MEIJER, Julia, NL-2333 CN Leiden (NL)
(74) Representative: Beslier, Victor
(86) International application number: PCT/EP2009/058761
(87) International publication number: WO 2010/006989

(56) References cited:
- WO-A-2008/081025
- SUBRAMANIAN SHYAMSUNDAR ET AL: "Scaleable production of adenoviral vectors by transfection of adherent PER.C6 cells." BIOTECHNOLOGY PROGRESS 2007 SEP-OCT, vol. 23, no. 5, September 2007 (2007-09), pages 1210-1217, XP002510292 ISSN: 8756-7938

## Description

The invention relates to the field of biotechnology. In particular, it relates to culturing human cells. More in particular it relates to adherently culturing PER.C6 cells.

### Background of the invention

Mammalian cells are either cultured adherently or in suspension, depending on the cell type. For scalability reasons, the trend is to adapt adherent cells to suspension cultures. The ability to adapt many cell types to suspension culture and the use of polymeric additives to reduce shear damage have enabled the widespread application of suspension culture, which is the system of choice for large scale applications (Chu and Robinson, 2001).

Scale up of adherent cells is far more difficult to achieve and has given rise to a wide range of alternative culture systems. One particular system that allows for scale-up of adherent cells are microcarrier cultures. The advantage of this methodology is that the cells, when growing on small carriers, can be treated as a suspension culture with all the advantages of large unit scale-up, homogeneity and easily controlled environmental conditions (Griffiths, 2001). Additionally, microcarrier cultures offer a high surface-to-volume ratio which leads to high cell density yields and a potential for obtaining highly concentrated cell products.

Several advances have been made in microcarrier technology. Charge group capacity and bead size of microcarriers have been optimized to enhance cell growth. Coating materials like collagen/gelatin, glass, ProNectin and Poly-Lysine have been applied to microcarrier surfaces. More carrier matrix materials have been introduced, including DEAE dextran, collagen/gelatin, glass, polystyrene, polyacrylamide, and cellulose (Kong et al, 1999).

Adherent cell cultures are widely used in the bio pharmaceutical industry, e.g. for the production of recombinant blood coagulation factors (e.g. US 2006/0194289) or the production of vaccines against Influenza (Brands et al, 1999). Different cell lines are used in these processes, including CHO, BHK, MDCK and Vero (Brühl et al, 2001; Rourou et al, 2007).

A large number of microcarriers are commercially available and the capability of cells to successfully proliferate on microcarriers must be tested for each cell line.

The human cell line PER.C6, which is used for the production of antibodies, vaccines and recombinant proteins (Jones et al, 2003; WO 00/63403; WO 01/38362), is particularly suitable for suspension culture (Yallop et al, 2005). However, for the production of some products, PER.C6 cells may preferably be cultured adherently.

Subramanian et al discloses the adherent culture of PER.C6 in T-flasks and the production of adenovirus particles after transfection. Subramanian et al also states that PER.C6 cells do not readily attach to cell culture substrata and PER.C6 cells had to be adapted to adherent culture conditions. WO 2008/081025 describes the adherent culture of PER.C6 cells in roller bottles to produce recombinant Factor XI. Although PER.C6 cells have been cultured adherently in flasks at small scale, no scalable processes of adherent culture of PER.C6 cells have been described thus far.

The possibility to culture PER.C6 cells adherently combined with the ability to scale up the culture would broaden the range of applications for the PER.C6 cell line.

### Summary of the invention

It was surprisingly found that PER.C6 cells do not adhere and grow well on the majority of microcarriers currently commercially available except for positively charged microcarriers. In addition, PER.C6 cells can, surprisingly, be detached easily from these carriers. This enables PER.C6 cells to be subcultured from one bioreactor to another, which is an advantage for process scale-up.

The present invention provides a method for adherently culturing PER.C6 cells comprising: a) attaching said cells to the surfaces of one or more DEAE-coated microcarriers; and b) culturing said cells in culture medium.

In other embodiments, the method of the invention further comprises a step of: c) detaching said cells from microcarriers and subsequently further culturing said cells.

In another embodiment of the invention said cells produce a product. In a preferred embodiment, said product is a protein. In another preferred embodiment said product is a blood coagulation factor.

It is also an object of the present invention to provide a method for producing a product by a PER.C6 cell, comprising the method of the invention and further comprising a step of: d) harvesting the product.

The invention also provides a cell culture comprising PER.C6 cells attached to DEAE-coated microcarriers, wherein said microcarriers are positively charged.

### Brief description of the Figures

Fig. 1: A representation of a culture, wherein cells did not attach to the carriers (refer to example 2).
Fig. 2: A representation of a culture, wherein cells were non-homogeneously distributed and tended to grow in large clumps attached to one or more carriers (refer to example 2).
Fig. 3: A representation of PER.C6 cells on Cytodex-1 microcarriers.
Fig. 4: Growth of PER.C6 cells on positively charged Cytodex-1 microcarriers.
Fig. 5: Metabolite concentrations in PER.C6 cells culture adherently growing on Cytodex-1 microcarriers.
Fig. 6: Metabolite concentrations in PER.C6 cells culture adherently growing on Cytopore-1 microcarriers.
Fig. 7: Metabolite concentrations in PER.C6 cells culture adherently growing on Cytopore-2 microcarriers.

### Detailed description of the invention

In accordance with these and other objects, the present invention provides methods for adherently culturing PER.C6 cells, comprising the steps of attaching said cells to the surfaces of positively charged microcarrier, culturing said cells in culture medium, detaching said cells from the microcarriers and further culturing said cells.

"Adherent cells" are cells, including mammalian cells, which attach to a surface, e.g. a tissue culture flask surface or a microcarrier particle surface, to replicate in tissue culture.

The cells of the invention are derived from E1-immortalized HER cells, such as PER.C6 cells. PER.C6 cells for the purpose of the present application have been described in US patent 5,994,128 and shall mean cells from an upstream or downstream passage or a descended of an upstream or downstream passage of cells as deposited under ECACC no. 96022940.

"Microcarriers" refers to particles, beads, or pellets useful for attachment and growth of adherent cells in culture. The microcarriers have the following properties: (a) They are small enough to allow them to be used in suspension cultures (with a stirring rate that does not cause significant shear damage to the microcarriers or the cells); (b) They are solid, or have a solid core with a porous coating on the surface; and (c) Their surfaces (exterior and interior surface in case of porous carriers) may be positively, negatively or not charged. Microcarriers may, for example, without limitation, be polystyrene, cellulose, polyethylene or dextran-based, which means that the matrix of the carriers can be made out of different materials e.g. polystyrene, cellulose, polyethylene or dextran. Additionally, microcarriers can be coated with an adhesion factor, such as gelatine, fibronectin, laminin, collagen, vitronectin or tenascin. These distinctive characteristics influence the binding capacity of different cell types to microcarriers and the ability of the cells to grow on the microcarriers.

In one aspect, the microcarriers have an overall particle diameter between about 150 and 350 µm. In one aspect, they have a positive charge density of between about 0.8 and 3.0 meq/g. The positive charge is provided by DEAE (N, N,-diethylaminoethyl) groups. The positive charge could also be provided by other groups, for instance it is known that DEAE groups are also used in anion exchange chromatography, where alternative positively charged groups such as quaternary ammonium groups are also used. It is therefore likely that such alternative positive groups could also be coupled to microcarriers and used according to the invention. Useful microcarriers are commercially available, for instance under the name Cytodex 1, Cytopore 1 and Cytopore 2 (from GE Healthcare). In certain embodiments, the microcarrier is a solid carrier (e.g. Cytodex-1). The carrier particle can also be a porous microcarrier (e.g. Cytopore 1 and Cytopore 2).

In the present invention, different cell lines were tested for their ability to adhere to several types of commercially available microcarriers. It was found that CHO cells can successfully be cultured on every type of microcarriers tested. In contrast, PER.C6 cells could not bind to any of these microcarriers, except for the positively charged microcarriers. Indeed, it is surprisingly found that PER.C6 cells can exclusively bind to positively charged microcarriers. The microcarriers used according to the method of the invention are therefore positively charged. The microcarriers, binding to PER.C6 cells, comprise a cross-linked dextran matrix which is substituted with positively charged N, N-diethylaminoethyl (DEAE) groups. The charged groups are distributed throughout the microcarrier matrix. A microcarrier comprising a cross-linked dextran matrix is referred to herein as "dextran-based". In other embodiments of the present invention, the positively charged microcarriers are "cellulose-based". In certain embodiments, the microcarriers are not coated with an adhesion factor.

The method of the invention allows adherent cells to be grown in conventional culture medium (GE Healthcare. Macrocarrier cell culture. Principles and methods. 18-1140-62). In preferred embodiments the medium contains serum. Serum, which is used to supplement the culture medium may come from a variety of sources and is used at concentrations ranging from 0.1% to 30% (v/v). The serum serves several functions. Firstly, it assists cells attach to the culture surface. Secondly, growth factors and hormones in the serum promote cell proliferation. The serum also has a protective effect on cells and products. A further function of the serum is to provide protease inhibitors that inactivate proteolytic enzymes used in routine sub-culturing.

The cells are cultured in conventional containers, such as shaker flasks, roller bottles, bioreactors and the like. It is within the knowledge of one skilled in the art to select the medium and optimal growth conditions, like supplements of the medium, serum, temperature, pH, stirring speed, microcarrier concentration, etc (GE Healthcare. Macrocarrier cell culture. Principles and methods. 18-1140-62). The microcarrier concentration in the start cell culture is preferably in the range of 1-2 g/L. The cell density of the culture preferably lies between 0.1 and 0.3 million viable cells per mL.

In order to scale up the process, it is preferred that the adherent cells can be detached from the carriers and subcultured in a greater volume. Adherent cells can for instance be detached from the carriers by the addition of proteolytic enzymes such as, without limitation, dispase, collagenase or trypsin to the medium. In certain embodiments, the proteolytic enzyme according to the invention is trypsin.

It was surprisingly found that, after the addition of trypsin, PER.C6 cells are easily detached from the positively charged microcarriers. This was highly unexpected considering the fact that these specific carriers are specified by the manufacturer to be strongly bound by cell lines and that these are therefore widely used as a carrier for end-stage production (GE Healthcare. Macrocarrier cell culture. Principles and methods. 18-1140-62).

According to a preferred embodiment of the invention the adherent cells can be detached from the carriers and transferred to a container with a higher volume. In a preferred embodiment, the cells are attached again to microcarriers and further cultured. This embodiment allows for the use of the adherent cells in an industrial process, which commonly requires several up-scaling steps.

Another aspect of the invention provides for a method for the production of a product by adherent PER.C6 cells, comprising the steps of attaching PER.C6 cells to the surface of microcarriers, culturing said cells in culture medium, wherein a product is released by said cells and subsequently harvesting the produced product.

According to one embodiment of the method the product produced by the adherent cells of the invention may be a protein as for example, without limitation, blood coagulation factors (e.g. Factor V, VIII, IX, X, XI, APC-resistant Factor V). For the production of proteins, the cells of the invention suitably comprise nucleic acid, encoding said proteins, in operable association with elements capable of driving expression of said proteins (see e.g. WO 00/63403).

Furthermore, the adherent cells of the invention can be used for the production of viruses by the cells, (see e.g. WO 01/38362), as some viruses may infect the cells more efficiently when the cells are adherent. Therefore a product produced by the methods of the invention in some embodiments may be a virus.

Methods for harvesting and further purifying the product are known to the skilled person in the art, which is able to design a suitable down stream process to recover said product from a cell suspension.

The present invention, for the first time, discloses PER.C6 cells bound to microcarriers. Hence, another aspect of the invention provides for a cell culture of adherent PER.C6 cells attached to microcarriers, wherein said microcarriers are positively charged. In certain embodiments, said microcarriers are dextran-based. In other embodiments, said microcarriers are cellulose-based. Such cell cultures can be used in processes according to the invention and for instance in scale-up.

According to the invention, said cell culture comprises culture medium and the microcarriers. It is within the knowledge of one skilled in the art to select the appropriate medium components to allow for maximal cell growth and product production (GE Healthcare. Macrocarrier cell culture. Principles and methods. 18-1140-62). In a certain embodiment the medium contains serum. Serum components can have a protective function for products that tend to be unstable e.g. antibodies or blood coagulation factors (e.g. Factor V, VIII, IX, X, XI, APC-resistant Factor V). The cell culture may further comprise product produced by the cells.

Having now generally described this invention, the same will be understood by reference to the following examples, which are provided herein for purposes of illustration only and are not intended to be limiting unless otherwise specified.

### EXAMPLES

### Example 1: Culture of CHO cells on commonly used microcarriers.

The ability of CHO cells to adhere to several microcarriers including Cytodex-1 and Cytodex-3 (GE healthcare); 2D Microhex and Biosilon (Nunc); HyQsphere CGEN 102-L, Pro-F 102-L, P102-L, Fact 102-L (Hyclone) was tested. The CHO cells were cultured on said microcarriers according to standard methods. The amount of carriers recommended by the manufacturers (1-20 g/L) was sterilized, washed with culture medium, and incubated at appropriate settings in spinner flasks for 30 min. The cells, which were obtained by trypsinization of T-Flask cultures, were inoculated at a density of 0.1-0.2x10E6 cells/mL, as recommended by the manufacturer. The cells were cultured in spinner flasks containing 75 ml of DMEM:F12 medium supplemented with 10% foetal bovine serum (FBS) in a humidified incubator incubator at 37°C and 5% CO₂ and 30 rpm. The culture of CHO-K1 cells, bound to the above-mentioned microcarriers, was performed successfully. In all cases, the cells attached well to the carriers, confluent growth was achieved on the carriers and homogeneous cultures were obtained.

### Example 2: Culture of PER.C6 cells on commonly used microcarriers.

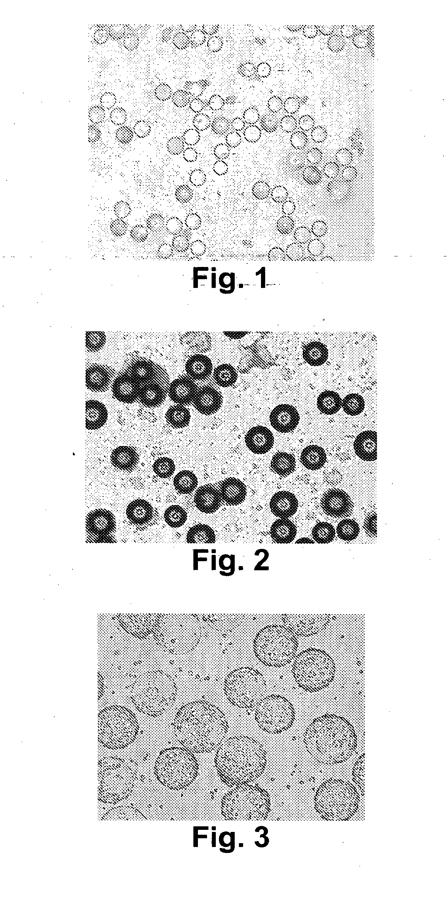

PER.C6 cells were tested on similar microcarriers as in the previous example (Table 1), according to the same protocol. It appeared that PER.C6 cells could not be cultured successfully on any of the following carriers: Cytodex-3 (GE healthcare); 2D Microhex and Biosilon (Nunc); HyQsphere CGEN 102-L, Pro-F 102-L, P102-L, Fact 102-L (Hyclone). In some cases the cells did not attach to the carrier (a representation of such a culture is shown in Fig. 1), and in other cases the cells were non-homogeneously distributed and tended to grow in large clumps attached to one or more carriers (a representation of such a culture is shown in Fig. 2).

### Example 3: Successful culture of PER.C6 cells on positively charged microcarriers.

PER.C6 cells were also tested on positively charged microcarrier (Cytodex-1) according to the protocol described in example 1. Cells grew to confluence in approximately 4-5 days (subconfluent culture at day 3 is shown on Fig. 3) and sufficient homogeneity of the culture (i.e. cells were evenly distributed over the microcarriers) was achieved. Four different PER.C6 cell lines expressing different glycoproteins, were tested. All were cultured successfully on positively charged microcarriers.

Since cells strongly adhere to positively charged microcarriers, the use of this type of carriers is recommended for final production culture by the manufacturer (GE Healthcare. Macrocarrier cell culture. Principles and methods. 18-1140-62). The possibility to detach viable cells from these microcarriers was tested and indeed, CHO cells could not be removed from the positively charged microcarriers.

Surprisingly, it was found that PER.C6 cells could be removed from the positively charged carriers by trypsinisation.

**Table 1. Summary of the results of examples 1, 2 and 3.**

| **Description microcarrier** | | **In-house results Crucell** | |
|---|---|---|---|
| **Name** | **Charge** | **PER.C6** | **CHO** |
| **2D Microhex** | Yes (-) | - | + |
| **Cytodex-1** | Yes (+) | + | + |
| **Cytodex-3** | No | - | + |
| **Biosilon** | Yes (-) | - | + |
| **HyQSphere CGEN 102L** | No | - | + |
| **HyQSphere Pro-F 102L** | No | - | + |
| **HyQSphere P102L** | No | - | + |

In general, it can be concluded that PER.C6 cells do not grow well on the majority of the microcarriers currently commercially available except for the positively charged microcarriers. In addition, PER.C6 cells can be removed from these carriers by trypsinisation. This enables subculturing and therefore scaling-up the process, which is required for an industrial process.

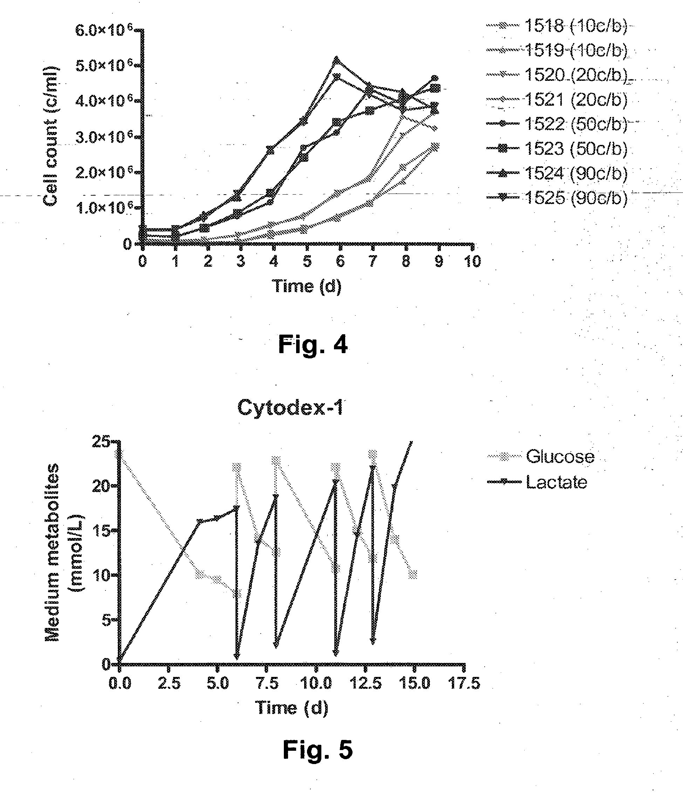

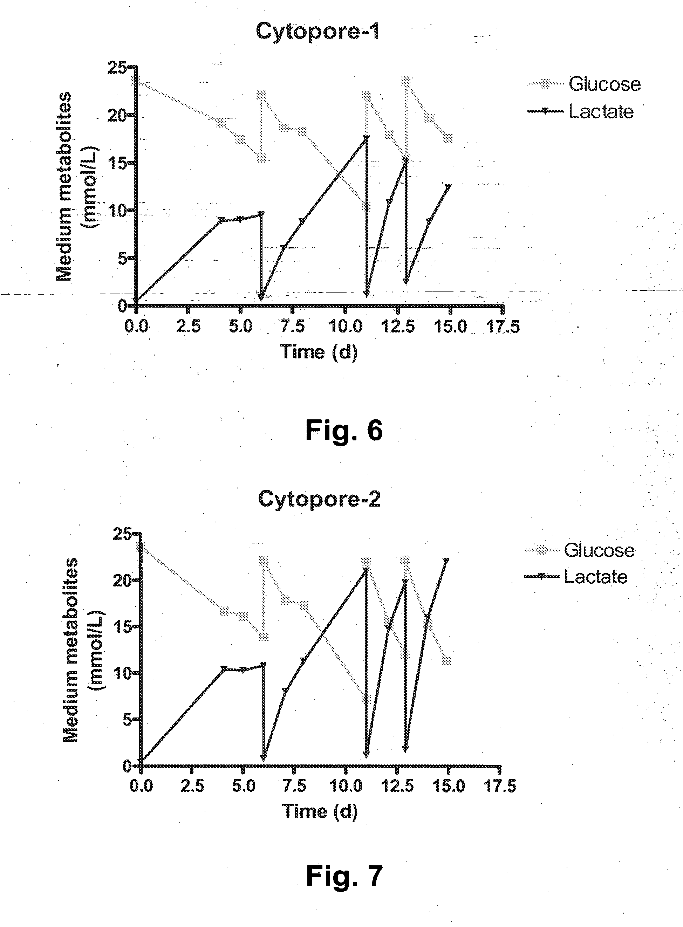

Besides the solid positively charged microcarrier Cytodex-1, two macroporous positively charged microcarriers (Cytopore-1 and Cytopore-2) were tested and it was shown that PER.C6 cells could be successfully cultured on these alternative positively charged microcarriers. Figs. 5, 6 and 7 show the glucose consumption and lactate production of PER.C6 cells adherently growing on positively charged macroporous microcarriers. The decreasing glucose concentration and increasing lactate production after each passage demonstrate that PER.C6 cells were cultured successfully on these microcarriers.

### Example 4: Batch culture of PER.C6 cells on positively charged carriers in spinners.

In this example, PER.C6 cells were cultured on positively charged carriers in spinner flasks. Cells were inoculated at a density of 0.1- 0.2x10E6 cells/mL in DMEM medium supplemented with 10% FBS. At inoculation, each bead contained between 10 and 90 cells as shown on Fig. 4. After 9 days, cell concentrations of approximately 5*10E6 vc/mL were reached, with approximately 1000 cells/bead (confluent culture). Successful cell cultures were obtained in wide ranges of the standard operating parameters such as stirrer speed (20 - 80 RPM) and working volume (150-250mL).

### Example 5: Batch culture of PER.C6 cells on positively charged carriers in Bioreactors.

PER.C6 cells were cultured on positively charged carriers in 2L bioreactors. The cells were cultured successfully. Cells attached well to the carriers, confluent growth was achieved on the carriers and homogeneous cultures were obtained.

### Example 6: Production of recombinant proteins by PER.C6 cells on positively charged carriers.

Production levels of several products produced by PER.C6 cells on positively charged microcarriers (in spinner flask) were measured. We found that the production levels of these proteins were in the range of levels previously obtained in reference systems (T-Flask). The production levels of Factor V proteins on positively charged carriers (in spinner flask) were between 1000-2000 ng/mL compared to 1500-3000 ng/mL in a T-Flask. The production levels of Factor XI proteins on the same microcarriers (in spinner flask) were between 7000-12000 ng/mL compared to about 15000 ng/mL in a T-Flask.

### Example 7: Anticipated process.

Based on the described results, the following process is proposed. Precultures of adherent PER.C6 cells are scaled up to production scale using Cytodex-1 microcarriers. Subsequently, the production (of e.g. recombinant proteins) is performed in one or multiple (e.g. 3-6) repeated batch cultures with medium exchange. For this final production step the Cytodex-1 microcarriers are used. Alternatively, also macroporous microcarriers may be used for this step. This may be advantageous since high cell densities can be reached, and we have observed that growth of PER.C6 on these carriers was promising. However, because cells cannot be removed from these carriers they are not suitable for the intermediate preculture/ scale-up steps.

### References

Brands et al. Influvac: A safe madin darby canine kidney (MDCK) cell culture-based influenza vaccine in Brown F, Robertson JS, Schild GC, Wood JM: Inactivated influenza vaccines prepared in cell culture, Dev Biol Stand. Basel, Karger, 1999, vol 98, pp 93-100.

Brühl P, Kerschbaum A, Kistner O, Barrett N, Dorner F, Gerencer M. Humoral and cell-mediated immunity to Vero cell-derived influenza vaccine. Vaccine 19 (2001) 1149-1158.

Chu L, and Robinson, D. Industrial choices for protein production by large-scale cell culture. Current Opinion in Biotechnology 2001, 12: 180-187.

Griffiths B. Scale-up of suspension and anchorage-dependent animal cells. Molecular Biotechnology, Volume 17, 2001.

Jones D, Kroos N, Anema R, van Montfort B, Vooys A, van der Kraats S, van der Helm E, Smits S, Schouten J, Brouwer K, Lagerwerf F, van Berkel P, Opstelten DJ, Logtenberg T, Bout A. High-level expression of recombinant IgG in the human cell line PER.C6. Biotechnol Prog. 2003 Jan-Feb; 9(1):163-8.

Kong D, Chen M, Gentz R & Zhang J. Cell growth and protein formation on various microcarriers. Cytotechnology 29: 149-156, 1999.

Rourou S, Van der Ark A, Van der Velden T, Kallel H. A microcarrier cell culture process for propagating rabies virus in Vero cells grown in a stirred bioreactor under fully animal component free conditions. Vaccine 25 (2007) 3879-3889.

Subramanian Shyamsundar et al: "Scalable production of adenoviral vectors by transfection of adherent PER.C6 cells." BIOTECHNOLOGY PROGRESS, vol. 23, no. 5.

Yallop C, Crowley J, Cote J, Hegmans-Brouwer K, Lagerwerf F, Gagne R, Martin JC, Oosterhuis N, Opstelten DJ, Bout A. Per.C6 cells for the manufacture of biopharmaceutical proteins. Modern Biopharmaceuticals - Design, Development and Optimization. Vol. 3, 2005.

## Claims

1. A method for adherently culturing PER.C6 cells as deposited under ECACC no. 96022940 comprising:
a) attaching PER.C6 cells to DEAE-coated microcarriers; and
b) culturing said cells in culture medium.

2. A method according to claim 1, further comprising a step of:
c) detaching said cells from microcarriers and subsequently further culturing said cells.

3. A method according to any one of the preceding claims, wherein said microcarriers are dextran-based.

4. A method according to any one of the preceding claims, wherein said cells produce a product.

5. A method according to claim 4, wherein said product is a protein.

6. A method according to claim 4, wherein said product is a blood coagulation factor.

7. A method according to any one of claims 4, 5 or 6, further comprising a step of:
d) harvesting the product.

8. A culture comprising PER.C6 cells as deposited under ECACC no. 96022940 attached to DEAE-coated microcarriers.

## Patentansprüche

1. Verfahren zur adhärenten Kultivierung von PER.C6-Zellen, wie unter ECACC-Nr. 96022940 hinterlegt, umfassend:
a) Binden von PER.C6-Zellen an DEAE-beschichtete Mikroträger; und
b) Kultivieren der Zellen in Kulturmedium.

2. Verfahren nach Anspruch 1, in einem weiteren Schritt umfassend:
c) Ablösen der Zellen von Mikroträgern und anschließend weiteres Kultivieren der Zellen.

3. Verfahren nach einem der vorhergehenden Ansprüche, wobei die Mikroträger auf Dextran basieren.

4. Verfahren nach einem der vorhergehenden Ansprüche, wobei die Zellen ein Produkt produzieren.

5. Verfahren nach Anspruch 4, wobei es sich bei dem Produkt um ein Protein handelt.

6. Verfahren nach Anspruch 4, wobei es sich bei dem Produkt um einen Blutgerinnungsfaktor handelt.

7. Verfahren nach einem der Ansprüche 4, 5 oder 6, in einem weiteren Schritt umfassend:
d) Ernten des Produkts.

8. Kultur, umfassend an DEAE-beschichtete Mikroträger gebundene PER.C6-Zellen, wie unter ECACC-Nr. 96022940 hinterlegt.

## Revendications

1. Méthode de culture de manière adhérente de cellules PER.C6 telles que déposées sous le numéro ECACC 96022940, comprenant :
a) la fixation des cellules PER.C6 à des micro-supports revêtus de DEAE ; et
b) la culture desdites cellules dans un milieu de culture.

2. Méthode selon la revendication 1, comprenant en outre une étape consistant à :
c) détacher lesdites cellules des micro-supports et cultiver ensuite davantage lesdites cellules.

3. Méthode selon l'une quelconque des revendications précédentes, dans laquelle lesdits micro-supports sont à base de dextrane.

4. Méthode selon l'une quelconque des revendications précédentes, dans laquelle lesdites cellules produisent un produit.

5. Méthode selon la revendication 4, dans laquelle ledit produit est une protéine.

6. Méthode selon la revendication 4, dans laquelle ledit produit est un facteur de coagulation sanguine.

7. Méthode selon l'une quelconque des revendications 4, 5 ou 6, comprenant en outre une étape consistant à :
d) récupérer le produit.

8. Culture comprenant des cellules PER.C6 telles que déposées sous le numéro ECACC 96022940, fixées à des micro-supports revêtus de DEAE.
